# EUROPÄISCHE PATENTSCHRIFT

(11) **EP 0 797 563 B1**
(45) Veröffentlichungstag und Bekanntmachung des Hinweises auf die Patenterteilung: **14.04.1999**
(21) Anmeldenummer: 95941663.7
(22) Anmeldetag: 04.12.1995
(51) Int. Cl.: C07C 69/52, C10M 105/38, A61K 7/40

(54) **SYNTHETISCHE ESTER AUS ALKOHOLEN UND FETTSÄUREGEMISCHEN AUS ÖLSÄUREREICHEN, STEARINSÄUREARMEN PFLANZENÖLEN**
SYNTHETIC ESTERS FROM ALCOHOLS AND FATTY ACID MIXTURES OF VEGETABLE OILS HIGH IN OLEIC ACID AND LOW IN STEARIC ACID
ESTERS SYNTHETIQUES CONSTITUES D'ALCOOLS ET DE MELANGES D'ACIDES GRAS D'HUILES VEGETALES RICHES EN ACIDE OLEIQUE ET PAUVRES EN ACIDE STEARIQUE

(30) Priorität: 12.12.1994 DE 4444137
(43) Veröffentlichungstag der Anmeldung: 01.10.1997
(73) Patentinhaber: Henkel Kommanditgesellschaft auf Aktien, 40191 Düsseldorf (DE)
(72) Erfinder: BONGARDT, Frank, D-40670 Düsseldorf (DE); BOSSMANN, Britta, D-40699 Erkrath (DE); WESTFECHTEL, Alfred, D-40723 Hilden (DE); GIEDE, Wolfgang, D-40764 Langenfeld (DE)
(86) Internationale Anmeldenummer: EP9504767
(87) Internationale Veröffentlichungsnummer: WO9618598

(56) Entgegenhaltungen:
- EP-A- 0 414 384
- DE-A- 2 231 162

## Beschreibung

Die Erfindung betrifft synthetische Ester aus Alkoholen und Fettsäuregemischen die mindestens 85 Gew.% Ölsäure und 0,5 bis 2,5 Gew.% Stearinsäure enthalten sowie deren Verwendung als Schmiermittel, Hydrauliköl und für kosmetische Zwecke.

### Stand der Technik

Mit dem Ziel einer vermehrten Nutzung nachwachsender Rohstoffe sind in neuerer Zeit verschiedene Ölpflanzen durch züchterische Maßnahmen bezüglich der Zusammensetzung ihrer Fettsäuregemische so verändert worden, daß sie für industrielle Zwecke besser geeignet sind. Beispiele dafür sind Raps, dessen Gehalt an Erucasäure abgesenkt worden ist, aber auch Raps mit einem erhöhten Erucasäuregehalt und Sonnenblumen, deren Öle einen durch Senkung des Linolsäureanteils angehobenen Ölsäuregehalt aufweisen.

Die gezielte Veränderung von Ölpflanzen zur Steigerung des ölsäuregehalts unter Senkung des Linolsäuregehalts ist in US-A-4 627 192 beschrieben. Die Zusammensetzung des "High Oleic Sunflower"-Öls wird z.B. in J. Amer. Oil Chem. Soc. 63, 1062 (1986) referiert. Der Gehalt an Ölsäure liegt bei etwa 80 bis 86 %, an Linolsäure bei 4 bis 8 %, an Stearinsäure bei 3 bis 5 %.

Die aus pflanzlichen oder tierischen Quellen gewonnenen Fette und Öle werden zu oleochemischen Rohstoffen verarbeitet. Wegen der C-Kettenverteilung der Fettsäuren, die in Fetten und Ölen auf natürlicher Basis immer vorliegt, erhält man dabei keine reinen Stoffe, sondern es fallen Gemische an, die Fettsäuren einer homologen Reihe enthalten.

Natürliche Triglyceride sind gegen Hydrolyse oder gegen oxidativen Abbau so wenig stabil, daß sie als Schmiermittel für viele Zwecke nicht verwendet werden können. Synthetische Ester der aus ihnen durch Spaltung gewonnenen Fettsäuregemische besitzen überlegene anwendungstechnische Eigenschaften.

Eine wichtige Anforderung an ein Schmiermittel auf Esterbasis ist ein möglichst niedriger Pourpoint nach DIN ISO 3016. Eine Möglichkeit, Schmiermittel mittlerer Viskosität und niedrigem Cloud-Point und Pour-Point zu erhalten, stellen Ester des Trimethylolpropans mit "Ölsäure" dar. Wegen der bereits erwähnten C-Kettenverteilung stellt die "Ölsäure" keinen Reinstoff, sondern eine Mischung verschiedener Fettsäuren dar.

Technische Ölsäure wird aus Talg gewonnen. Um einen genügend niedrigen Trübungspunkt, gemessen nach der Methode der Deutschen Gesellschaft für Fettforschung D-III 3 [79] zu erhalten, sind besondere Maßnahmen zur Reinigung des Fettsäuregemisches erforderlich, und zwar wird in einer sogenannten Umnetztrennung der größte Teil der gesättigten Fettsäuren abgetrennt. Dadurch wird der Trübungspunkt dieses Fettsäuregemisches auf Werte von 5 °C bis 10 °C abgesenkt. Das Gemisch mit einer Zusammensetzung von z.B. 67 % Ölsäure, 12 % Linolsäure und 2 % Stearinsäure kommt unter dem Namen Edenor^{R} TiO5 in den Handel und liefert bei Veresterung mit Trimethylolpropan Ester mit einem Cloud-Point von -20 °C und einem Pourpoint von -40 °C.

Die auf die beschriebene Weise nach dem Stand der Technik hergestellten Ester sind jedoch wegen des Gehalts an oxidationsempfindlicher Linolsäure bezüglich ihrer Stabilität gegen Oxidation nicht völlig zufriedenstellend. Auch Farbe und Geruch sind noch verbesserungswürdig.

Weitere Probleme entstehen durch Qualitätsschwankungen, da als Rohstoffquelle tierische Fette der unterschiedlichsten Herkunft verwendet werden. Erfahrungsgemäß kann das zu nicht vorhersehbaren Schwierigkeiten bei der Einhaltung der Spezifikation des Cloud-Points und des Pourpoints führen.

Zur Vermeidung dieser Probleme scheint es naheliegend zu sein, einen Rohstoff mit einem höheren Gehalt an Ölsäure zu verwenden, z.B. das eingangs beschriebene ölsäurereiche Sonnenblumenöl.

Die Verwendung eines Fettsäuregemisches aus Sonnenblumenöl neuer Züchtung (Ölsäuregehalt etwa 85 %, Linolsäuregehalt etwa 5 %, Stearinsäuregehalt etwa 4 %, Palmitinsäuregehalt etwa 4 %) führt jedoch nicht zum Ziel: der Cloud-Point des Trimethylolpropanesters liegt bei - 10 °C, der Pour-Point bei - 20 °C.

Das Umnetzverfahren bleibt aber auf Fettsäuregemische beschränkt, die noch einen relativ hohen Linolsäuregehalt haben. Der Trübungspunkt eines hochölsäurehaltigen Fettsäuregemisches aus neuer Sonnenblume kann deshalb nicht durch Umnetztrennung auf 5 °C abgesenkt werden, denn die reine Ölsäure hat schon einen Schmelzpunkt von 14 °C.

Hydrauliköle enthalten im allgemeinen kein Wasser. Durch Defekte oder im Dauerbetrieb kann es trotzdem zum Eindringen von Wasser ins Hydrauliköl kommen. Dieses Wasser darf nicht im Hydrauliköl emulgiert werden, sondern sollte sich möglichst rasch als zweite Phase abscheiden. Dieses sogenannte Demulgiervermögen von Hydraulikölen wird über die DIN 51 599 gemessen. Das Demulgiervermögen von Estern auf Basis der technischen Ölsäure ist noch verbesserungswürdig.

Die Aufgabe der Erfindung hat demnach darin bestanden, alternative Rohstoffe zur Herstellung von Estern auf Basis von technischer Ölsäure zu finden, mit denen ohne Abtrennung gesättigter Fettsäuren Ester von Alkoholen mit sicher reproduzierbarem, niedrigem Cloud-Point und Pour-Point, heller Farbe, guter Oxidationsbeständigkeit und gutem Demulgiervermögen erhältlich sind.

Es wurde überraschend gefunden, daß Fettsäuregemische aus dem Öl der ölsäurereichen, stearinsäurearmen Sonnenblume sich für diesen Zweck besonders gut eignen und eine Reihe unerwarteter Vorteile, z.B. beim Demulgiervermögen, aufweisen.

### Beschreibung der Erfindung

Gegenstand der Erfindung sind synthetische Ester aus
a) Fettsäuregemischen,
b) Alkoholen, sowie
c) gewünschtenfalls polyfunktionelle Carbonsäuren, bei denen die Fettsäuregemische wenigstens 85 Gew.% an Ölsäure und 0,5 bis 2,5 Gew.% an Stearinsäure - jeweils bezogen auf das Gemisch - enthalten.

Ein weiterer Gegenstand der Erfindung ist die Verwendung der Ester für Schmierstoffe, Hydrauliköle und kosmetische Zwecke.

Durch pflanzenzüchterische Methoden ist es möglich, Ölpflanzen mit besonders niedrigem Gehalt an Stearinsäure zu erhalten, z.B. die in der EP-A1-0 496 504 beschriebene Sonnenblume mit einem auf maximal 6 Gew.% abgesenktem Gehalt an gesättigten Fettsäuren. Sonnenblumen nach diesem Patent ergeben in einer bevorzugten Form ein fettes Öl mit einem Gehalt von 1 Gew.% oder weniger an Stearinsäure und nicht mehr als 4 Gew.% an Palmitinsäure.

In einer bevorzugten Ausführungsform der Erfindung werden Fettsäuregemische zur Herstellung von Estern verwendet, die aus diesem "high oleic, low stearic" Sonnenblumenöl gewonnen wurden.

Zur Isolierung der Fettsäuregemische wird das Öl auf übliche Art unter Druck mit Wasser gespalten, das Glycerin abgetrennt und das Gemisch der freien Säuren isoliert.

Die Fettsäurezusammensetzung ist in Tabelle 1 dargestellt.

**Tabelle 1:**

| Zusammensetzung des "high oleic, low stearic" Sonnenblumenöls | |
|---|---|
| Fettsäurekomponente | Anteil Gew. % |
| Palmitinsäure | 2 bis 5 |
| Stearinsäure | 0,5 bis 2,5 |
| Ölsäure | 85 bis 95 |
| Linolsäure | 2 bis 8 |

Das durch Spaltung des Öls gewonnene Fettsäuregemisch hat eine Zusammensetzung von maximal 6 Gew.% an gesättigten Fettsäuren (Palmitin- und Stearinsäure), bevorzugt von 3,6 bis 5,0 Gew.% an gesättigten Fettsäuren.

Von diesen gesättigten Fettsäuren sind maximal bis zu 2,5 Gew.%, bevorzugt bis zu maximal 1,5 Gew.% Stearinsäure.

Falls gewünscht kann die Palmitinsäure durch fraktionierte Destillation abgetrennt werden, um den Reinheitsgrad der Ölsäure weiter zu erhöhen.

Als Alkohole kommen monofunktionelle und polyfunktionelle Alkohole in Frage.

Geeignete monofunktionelle Alkohole sind aliphatische oder cycloaliphatische, gesättigte oder ungesättigte Alkohole mit 2 bis 22 C-Atomen.

Bevorzugte Alkohole sind n-Propanol, i-Propanol, n-Butanol, i-Butanol, Hexanol, Octanol, Decanol, Kokosfettalkohol, Stearylalkohol, 2-Ethylhexanol, i-Nonanol, i-Tridecylalkohol und Oleylalkohol.

Die vorstehend beschriebenen Alkohole können einzeln oder in der Form von Gemischen eingesetzt werden.

Es ist selbstverständlich auch möglich, anstelle der Alkohole deren Umsetzungsprodukte mit Alkylenoxiden, vorzugsweise deren Ethoxylate oder Propoxylate einzusetzen.
Jede Hydroxylgruppe kann mit 1 bis 100, bevorzugt 1 bis 30, besonders bevorzugt 1 bis 10 mol Alkylenoxid umgesetzt worden sein.

In einer bevorzugten Ausführungsform der Erfindung stellen die Alkohole polyfunktionelle Alkohole, sogenannte Polyole dar. Darunter sind Alkohole zu verstehen, die mehr als eine Hydroxylgruppe im Molekül aufweisen.

In einer bevorzugten Ausführungsform enthalten die Polyole 2-6 Hydroxylgruppen. Bevorzugt enthalten die Polyole 2 bis 12 C-Atome.

Geeignete Polyole sind z.B. Diole wie Ethylenglykol, 1,2-Propandiol, 1,3-Propandiol, 1,4-Butandiol, 1,6-Hexandiol, 1,8-Octandiol, 1,10-Decandiol, 1,12-Octadecandiol, Diethylenglykol, Triethylenglykol oder Bishydroxymethylcyclohexan.

Geeignete höherfunktionelle Polyole sind z.B. Glycerin, Polyglycerin, Sorbit oder Mannit.

In einer besonders bevorzugten Ausführungsform der Erfindung werden Polyole mit einer sogenannten Neo-Struktur eingesetzt, d.h. Polyole, die sich vom Neopentan ableiten. Solche Polyole zeichnen sich dadurch aus, daß sie an den Kohlenstoff-Atomen, die dem die Hydroxylgruppe tragenden Kohlenstoff-Atom benachbart sind, keinen Wasserstoff aufweisen.

Bevorzugte Beispiele solcher Polyole sind Neopentylglykol, Trimethylolethan, Trimethylolpropan, Ditrimethylolpropan, Pentaerythrit, Dipentaerythrit und Tripentaerythrit.

Es ist selbstverständlich auch möglich, anstelle der Polyole deren Umsetzungsprodukte mit Alkylenoxiden, vorzugsweise deren Ethoxylate oder Propoxylate einzusetzen.
Jede freie Hydroxylgruppe im Polyol kann mit 1 bis 100, bevorzugt 1 bis 30, besonders bevorzugt 1 bis 10 mol Alkylenoxid umgesetzt worden sein.

Die vorstehend beschriebenen Polyole können einzeln oder in der Form von Gemischen eingesetzt werden.
Selbstverständlich können auch Mischungen von monofunktionellen Alkoholen und Polyolen eingesetzt werden.

In einer bevorzugten Ausführungsform sind die Polyole vollständig oder nahezu vollständig mit den erfindungsgemäßen Fettsäuregemischen verestert.

Aber auch Partialester der vorstehend genannten Polyole, bei denen im Mittel 16 bis 80 mol% der Hydroxylgruppen verestert sind, gehören zum Umfang der Erfindung. Beispiele bevorzugter Partialester sind Glycerin, verestert mit einem oder zwei Mol eines erfindungsgemäßen Fettsäuregemisches, Trimethylolpropan, verestert mit einem oder zwei Mol eines erfindungsgemäßen Fettsäuregemisches oder Pentaerythrit, verestert mit einem oder zwei Mol eines erfindungsgemäßen Fettsäuregemisches.

Die erfindungsgemäßen Partialester zeichnen sich dadurch aus, daß die Ablagerungen, die sich in Partialestern auf Basis von technischer Ölsäure mit der Zeit bilden, nicht mehr auftreten oder zumindest stark verringert sind.

Neben den einfachen Estern der monofunktionellen Fettsäuren mit den Polyolen können durch anteiligen Einsatz von polyfunktionellen Carbonsäuren bei der Veresterung auch die an sich bekannten komplexen Ester hergestellt werden.
Die polyfunktionellen Carbonsäuren stellen bevorzugt Di- und/oder Tricarbonsäuren mit 2 bis 54 C-Atomen dar.

Bevorzugt sind z.B. Bernsteinsäure, Adipinsäure, Sebacinsäure, Isomere der Phthalsäure oder Dimerfettsäure. Eine besonders geeignete trifunktionelle Carbonsäure ist die Trimerfettsäure, die beispielsweise durch Aufarbeitung des Destillationsrückstands der durch Dimerisierung von Tallölfettsäure erhaltenen Dimerfettsäure erhältlich ist.

Selbstverständlich können auch Gemische der einzelnen Di- und/oder Tricarbonsäuren verwendet werden.

Bei der Herstellung der synthetischen Ester liegt das Äquivalent-Verhältnis OH:COOH im allgemeinen im Bereich von 1,2:1 bis 0,9:1, bevorzugt im Bereich von 1,05:1 bis 0,95:1, wenn vollständige Veresterung angestrebt wird.

Bei der anteiligen Verwendung von polyfunktionellen Carbonsäuren liegt das Äquivalentverhältnis von COOH aus polyfunktionellen Carbonsäuren zu COOH aus monofunktionellen Carbonsäuren im Bereich von 0,05 bis 0,5, bevorzugt von 0,01 bis 0,2, besonders bevorzugt im Bereich von 0,05 bis 0,15.

Die Herstellung kann nach den üblichen Methoden zur Herstellung von Estern erfolgen. Beispielsweise können die Polyole und die Fettsäuren in einem Reaktionsgefäß unter Rühren auf Temperaturen von 100 bis 250 °C erwärmt werden, bis die Veresterungsreaktion unter Abspaltung von Wasser einsetzt.
Die Abtrennung des Wassers kann durch azeotrope Entfernung mit einem Schleppmittel erleichtert oder durch ein durchgeleitetes Inertgas beschleunigt werden.
Zur Beschleunigung der Veresterungsreaktiion können übliche Katalysatoren, z.B. p-Toluolsulfonsäure oder Zinnverbindungen wie Zinnoxalat oder Zinnoctoat, aber auch reines Zinn in Form von Zinnschliff zugegeben werden.
Die Veresterungsreaktion wird im allgemeinen so lange geführt, bis kein weiteres Wasser mehr abgetrennt wird.

Selbstverständlich können die erfindungsgemäßen Ester auch durch eine Umesterungsreaktion hergestellt werden, z.B. aus den Methylestern der Fettsäuren und den Polyolen unter Abtrennung von Methanol. Zur Beschleunigung dieser Umesterung können übliche Katalysatoren wie Natriummethylat, Zn-Seifen oder Titanverbindungen wie Titantetraisopropylat zugegeben werden.

Die Herstellung der komplexen Ester kann so erfolgen, daß Polyol, Fettsäure und polyfunktionelle Carbonsäure in einer Einstufenreaktion umgesetzt werden. Es ist aber auch möglich, die Reaktion in mehreren Stufen zu führen, z.B. indem man zunächst einen Teil oder die gesamte Menge an Monocarbonsäure mit dem Polyol umsetzt und nach Beendigung dieser Reaktion die verbliebenen Hydroxlygruppen mit der polyfunktionellen Carbonsäure bzw. ihrem Gemisch mit verbliebener Monocarbonsäure umsetzt.
Auch die Herstellung der komplexen Ester kann über eine Umesterung erfolgen.

Im Anschluß an die Herstellung kann - falls erforderlich - eine Reinigung der erfindungsgemäßen Ester erfolgen, z.B. durch Abdestillieren von Verunreinigungen, bevorzugt unter vermindertem Druck, durch Waschen mit verdünnten Laugen oder durch adsorptive Behandlungen z.B. mit Bleicherde oder mit Calciumhydroxid.

Hydrauliköle sind Flüssigkeiten für die hydrostatische Kraftübertragung. Es wurde gefunden, daß Hydrauliköle, die die erfindungsgemäßen Ester enthalten, insbesondere Ester auf Basis von polyfunktionellen Alkoholen und besonders bevorzugt Ester des Trimethylolpropans, besonders gute Eigenschaften besitzen.

Ein weiterer Gegenstand der Erfindung sind demnach Hydrauliköle, enthaltend synthetische Ester aus Fettsäuregemischen mit wenigstens 85 Gew.% an Ölsäure und 0,5 bis 2,5 Gew.% an Stearinsäure - jeweils bezogen auf das Gemisch - und Alkoholen, sowie gewünschtenfalls polyfunktionellen Carbonsäuren.

Die erfindungsgemäßen Hydrauliköle enthalten mindestens 75 Gew.%, bevorzugt mindestens 95 Gew.% der erfindungsgemäßen Ester. Weiterhin können sie noch weitere, für diese Verwendung an sich bekannte Zusätze enthalten, z.B. Antischaummittel, Anti-Stick-Slip-zusätze, Korrosionsinhibitoren, Oxidationsinhibitoren, Pour-Point-Verbesserer, Verschleißschutz-Additive, Detergentien und/oder Emulgatoren.

Die Hydrauliköle können auch noch - wenngleich nicht bevorzugt - Mineralöl in geringen Mengen enthalten.

Die Hydrauliköle finden wegen ihrer guter biologischen Abbaubarkeit vor allem dort Verwendung, wo beim Einsatz gelegentlich Hydrauliköl in die Umwelt freigesetzt wird, also z.B. bei landwirtschaftlichen Maschinen.

Die erfindungsgemäßen synthetischen Ester, bevorzugt die von Polyolen, können als Schmiermittel oder Bestandteil von Schmiermitteln verwendet werden.

Die als Schmiermittel verwendeten erfindungsgemäßen Ester weisen im allgemeinen folgende Kennzahlen auf:
eine Hydroxylzahl von maximal 25, bevorzugt von maximal 5,
eine Säurezahl von maximal 2, bevorzugt von maximal 0,5,
einen Pourpoint von mindestens - 40 °C,
eine Farbzahl nach Lovibond 1 Zoll, bestimmt nach der Methode der Deutschen Gesellschaft für Fettforschung (DGF) C-V 4b [84] von maximal 7 gelb, bevorzugt maximal 5 gelb und maximal 1,5 rot, bevorzugt maximal 0,5 rot.

Entsprechend den Anforderungen an das Schmiermittel kann die Viskosität unterschiedliche Werte annehmen, für Ester des Trimethylolpropans liegt sie im Bereich von 40 bis 50 mm²/s bei 40 °C, bestimmt nach DIN 51 562.

Eine weitere Verwendungsmöglichkeit liegt auf dem Gebiet der Hydrauliköle.

Außerdem können die erfindungsgemäßen Ester wegen ihren guten Eigenschaften bezüglich Farbe, Geruch und niedrigem Cloud-Point Verwendung für kosmetische Zwecke finden, und zwar vor allem als Ölkörper.

### Beispiele

Alle prozentualen Angaben in den Beispielen verstehen sich, sofern nicht anders vermerkt, als Gewichtsprozent.

**Tabelle 2:**

| Vergleich der Fettsäuregemisches aus "high oleic, low stearic" Sonnenblume (Sb_{HO,LS}) und Edenor^{R}TiO5: | | |
|---|---|---|
| | Fettsäuregemische* | |
| | Sb_{HO,LS} erf.-gem. | TiO5 Vergleich |
| Palmitinsäure | 3,5 % | 5 % |
| | | |
| Stearinsäure | 1,0 % | 1 % |
| | | |
| Ölsäure | 91,0 % | 71 % |
| | | |
| Linolsäure | 4,2 % | 11 % |
| | | |
| Trübungspunkt (DGF D-III 3 [79]) | + 9 °C | + 5 °C |

| | | |
|---|---|---|
| * Die Werte addieren sich nicht zu 100 %, weil nur die wichtigsten Fettsäuren genannt sind. | | |

Aus der Tabelle geht hervor, daß der Trübungspunkt des Fettsäuregemisches aus "high oleic, low stearic" Sonnenblumenöl deutlich höher liegt als bei der technischen Ölsäure (Edenor^{R} TiO5).

### Ester mit Trimethylolpropan (Beispiel 1 und Vergleichsbeispiel 1)

Aus beiden Fettsäuregemischen wurden Ester des Trimethylolpropans hergestellt.

### Ansatz:

2,67 mol Trimethylolpropan
8,0 mol Fettsäuregemisch
0,3 Gew.% Zinn-II-oxalat, bezogen auf Fettsäuregemisch,
wurden in einem Reaktionsgefäß mit Rührer, Stickstoffeinleitung und Wasserabscheider bei Temperaturen von etwa 200 °C bis 220 °C solange verestert, bis kein Wasser mehr abgeschieden wurde.
Danach wurde 1 Gew.% Bleicherde (Tonsil) - bezogen auf Reaktionsansatz - zugegeben, 10 Minuten gerührt und abfiltriert.

**Tabelle 3:**

| Anwendungstechnische Eigenschaften von TMP-Estern auf Basis technischer Ölsäure und des Fettsäuregemisches aus "high oleic,low stearic" Sonnenblume | | |
|---|---|---|
| Eigenschaft | Sb_{HO,LS} erf.-gem. | TiO5 Vergleich |
| Viskosität nach DIN 51562, 40 °C | 50 mm²/s | 46 mm²/s |
| | | |
| Viskosität nach DIN 51562, 100 °C | 10 mm²/s | 9,5 mm²/s |
| | | |
| Viskositätsindex DIN ISO 2909 | 190 | 180 |
| | | |
| Cloudpoint DIN ISO 3015 | - 35° C | - 20° C |
| | | |
| Pour-Point DIN ISO 3016 | - 50° C | - 40° C |
| | | |
| Säurezahl DIN 53402 | 0,4 | 0,5 |
| | | |
| Hydroxylzahl DIN 53240 | 7 | 7 |
| | | |
| Farbzahl | gelb 3,2 | gelb 6,8 |
| Lovibond 1 Zoll DGF C-V 4b [84] | rot 0,4 | rot 1,5 |
| | | |
| Geruch | neutral | fettig, leicht ranzig |
| | | |
| Oxidationsstabilität Ranzimat | 13 h | 4,5 h |
| | | |
| Demulgiervermögen DIN 51381 | 20 min | > 60 min |

Die Oxidationstabilität wurde im Ranzimat-Gerät der Firma Metrohm gemessen. Angegeben wurde die Induktionsperiode, das ist die Zeit bis zum Ansteigen der Leitfähigkeit.

Der Vergleich der anwendungstechnischen Daten zeigt klare Vorteile hinsichtlich Cloud-point, Pour-Point, Oxidationstabilität und Demulgiervermögen für die erfindungsgemäßen Ester.

### Ester mit Decylalkohol (Beispiel 2 und Vergleichsbeispiel 2)

561 g (2 mol) Fettsäuregemisch wurden mit 364,6 g (2,3 mol) eines C₁₀ - C₁₈-Fettalkoholgemisches (Hydroxylzahl = 354) unter Zugabe von 0,032 g Zinn-II-oxid in einer Rührapparatur mit Wasserabscheider und Stickstoffeinleitung bei Temperaturen von 200 bis 220 °C verestert bis die Säurezahl auf einen Wert von 0,17 abgefallen war.
Zur Reinigung wurde das Produkt anschließend destilliert. Der Siedepunkt liegt bei 226 bis 230 °C bei 0,1 bis 0,09 mbar.

Bei Verwendung des Fettsäuregemisches aus "high oleic, low stearic"-Sonnenblumenöl erhält man einen Ester mit den Kennzahlen:

Säurezahl: 0,3; Verseifungszahl: 131; Hydroxylzahl: 3,5; Jodzahl: 59
Der Cloud-point liegt bei 0 bis -1 °C.

Der analog hergestellte Ester aus Edenor^{R} TiO5 hat einen Cloud-Point von + 5 °C.

### Ester mit 2-Ethylhezanol (Beispiel 3 und Vergleichsbeispiel 3)

283,3 g (1 mol) Fettsäuregemisch wurden mit 143,2 g (1,1 mol) 2-Ethylhexanol unter Zugabe von 0,013 g Zinn-II-oxid in einer Rührapparatur mit Wasserabscheider und Stickstoffeinleitung bei Temperaturen von 200 bis 220 °C verestert bis die Säurezahl auf einen Wert von 0,8 abgefallen war.
Zur Reinigung wurde das Produkt anschließend destilliert. Der Siedepunkt liegt bei 167 bis 186 °C bei 0,1 bis 0,09 mbar.
Bei Verwendung des Fettsäuregemisches aus "high oleic, low stearic"-Sonnenblumenöl erhält man einen Ester mit den Kennzahlen:

Säurezahl: 0,2; Verseifungszahl: 142; Hydroxylzahl: 3,7; Jodzahl: 65
Der Cloud-point liegt bei - 27 °C.

Der analog hergestellte Ester aus Edenor^{R} TiO5 hat einen Cloud-Point von - 16 °C.

### Ester mit Neopentylglykol (Beispiel 4)

425 g (1,5 mol) eines Fettsäuregemisches aus "high oleic, low stearic" Sonnenblumenöl wurden mit 87,2 g (0,837 mol) Neopentylglykol unter Zugabe von 0,05 g Zinn-II-oxid in einer Rührapparatur mit Stickstoffeinleitung und Wasserabscheider bei Temperaturen von 200 bis 240 °C verestert bis die Säurezahl auf einen Wert von < 3 abgefallen war. Danach wurde noch für 3 Stunden bei einem Vakuum von 20 Torr und einer Temperatur von 240 °C reagieren gelassen.
Zur Reinigung wurde das Produkt mit 5,3 g Bleicherde (Tonsil Standard) versetzt und 1 Stunde bei 90 °C gerührt. Anschließend wurde abfiltriert.

### Komplexester (Beispiel 4)

566,6 g (2 mol) eines Fettsäuregemisches aus "high oleic, low stearic" Sonnenblumenöl wurden mit 108,5 g (0,81 mol) Trimethylolpropan, 98%ig, und 92,9 g (0,159 mol) einer Dimerfettsäure (MG: 584,4) unter Zugabe von 0,22 g Zinn-II-oxalat in einer Rührapparatur mit Stickstoffeinleitung und Wasserabscheider bei Temperaturen von 200 bis 240 °C verestert bis die Säurezahl auf einen Wert von 2 abgefallen war. Danach wurde noch für 3 Stunden bei einem Vakuum von 20 bis 2 Torr und einer Temperatur von 240 °C weiter verestert, bis die Säurezahl auf 0,5 gefallen war.

Zur Reinigung wurde das Produkt mit 3,2 g Bleicherde (Tonsil Standard) versetzt und 1 Stunde bei 90 °C gerührt. Anschließend wurde abfiltriert.

## Patentansprüche

1. Synthetische Ester aus
a) Fettsäuregemischen,
b) Alkoholen, sowie
c) gewünschtenfalls polyfunktionelle Carbonsäuren,
dadurch gekennzeichnet, daß die Fettsäuregemische wenigstens 85 Gew.-% an Ölsäure und 0,5 bis 2,5 Gew.-% an Stearinsäure - jeweils bezogen auf das Gemisch - enthalten.

2. Synthetische Ester nach Anspruch 1, dadurch gekennzeichnet, daß der Stearinsäuregehalt in den Fettsäuregemischen zwischen 0,5 und 1,5 Gew.-% - bezogen auf das Gemisch - liegt.

3. Synthetische Ester nach den Ansprüchen 1 und 2, dadurch gekennzeichnet, daß "High Oleic, Low Stearic Sunflower" als Ölsaat zur Gewinnung der Fettsäuregemische verwendet wird.

4. Synthetische Ester nach den Anprüchen 1 bis 3, dadurch gekennzeichnet, daß die Alkohole Polyole darstellen.

5. Synthetische Ester nach den Ansprüchen 1 bis 4, dadurch gekennzeichnet, daß die Polyole 2 bis 6 Hydroxylgruppen enthalten.

6. Synthetische Ester nach den Ansprüchen 1 bis 5, dadurch gekennzeichnet, daß dic Polyole am Kohlenstoff-Atom, das dem die Hydroxylgruppe tragenden Kohlenstoff-Atom benachbart ist, keinen Wasserstoff aufweisen.

7. Synthetische Ester nach den Ansprüchen 1 bis 6, dadurch gekennzeichnet, daß die Polyole Neopentylglykol, Trimethylolethan, Trimethylolpropan, Ditrimethylolpropan, Pentaerythrit, Dipentaerythrit oder Tripentaerythrit oder deren Gemische darstellen.

8. Synthetische Ester nach den Ansprüchen 4 bis 7, dadurch gekennzeichnet, daß die Ester der Polyole Partialester darstellen, bei denen im Mittel 16 bis 80 mol% der Hydroxylgruppen des Polyols verestert sind.

9. Synthetische Ester nach den Ansprüchen 1 bis 7, dadurch gekennzeichnet, daß die polyfunktionellen Carbonsäuren Di- und/oder Tricarbonsäuren mit 2 bis 54 C-Atomen darstellen.

10. Synthetische Ester nach Anspruch 9, dadurch gkennzeichnet, daß die Di- und/oder Tricarbonsäuren Bersteinsäure, Adipinsäure, Sebacinsäure, Isomerer der Phthalsäure, Dimerfettsäure oder Trimerfettsäure oder deren Gemische darstellen.

11. Synthetische Ester nach den Ansprüchen 9 und 10, dadurch gekennzeichnet, daß das Äquivlentverhältnis OH:COOH im Bereich von 1,2:1 bis 0,9:1, bevorzugt im Bereich von 1,05:1 bis 0,95:1 liegt.

12. Synthetische Ester nach den Ansprüchen 9 bis 11, dadurch gekennzeichnet, daß das Verhältnis von COOH aus polyfunktionellen Carbonsäuren zu COOH aus monofunktionellen Carbonsäuren im Bereich von 0,005 bis 0,5, bevorzugt von 0,01 bis 0,2, besonders bevorzugt von 0,05 bis 0,15, liegt.

13. Hydrauliköle, enthaltend synthetische Ester aus
a) Fettsäuregemischen
b) Alkoholen, sowie
c) Gewünschtenfalls polyfunktionellen Carbonsäuren, dadurch gekennzeichnet, daß die Fettsäuregemische wenigstens 85 Gew.-% an Ölsäure und 0,5 bis 2,5 Gew.-% an Stearinsäure - jeweils bezogen auf das Gemisch - enthalten.

14. Verwendung der synthetischen Ester nach den Ansprüchen 1 bis 7 und 9 bis 12 als Schmiermittel.

15. Verwendung der synthetischen Ester nach den Ansprüchen 1 bis 7 und 9 bis 12 als Hydrauliköle.

16. Verwendung der synthetischen Ester nach den Ansprüchen 1 bis 12 für kosmetische Zwecke, bevorzugt als Ölkörper.

## Claims

1. Synthetic esters of
a) fatty acid mixtures,
b) alcohols and
c) if desired, polybasic carboxylic acids,
characterized in that the fatty acid mixtures contain at least 85% by weight of oleic acid and 0.5 to 2.5% by weight of stearic acid, based on the mixture.

2. Synthetic esters as claimed in claim 1, characterized in that the stearic acid content of the fatty acid mixtures is between 0.5 and 1.5% by weight, based on the mixture.

3. Synthetic esters as claimed in claims 1 and 2, characterized in that high-oleic, low-stearic sunflower is used as the oil seed for obtaining the fatty acid mixtures.

4. Synthetic esters as claimed in claims 1 to 3, characterized in that the alcohols are polyols.

5. Synthetic esters as claimed in claims 1 to 4, characterized in that the polyols contain 2 to 6 hydroxyl groups.

6. Synthetic esters as claimed in claims 1 to 5, characterized in that the polyols do not have any hydrogen at the carbon atom adjacent the carbon atom carrying the hydroxyl group.

7. Synthetic esters as claimed in claims 1 to 6, characterized in that the polyols are neopentyl glycol, trimethylol ethane, trimethylol propane, ditrimethylol propane, pentaerythritol, dipentaerythritol or tripentaerythritol or mixtures thereof.

8. Synthetic esters as claimed in claims 4 to 7, characterized in that the esters of the polyols are partial esters in which on average 16 to 80 mole-% of the hydroxyl groups of the polyol are esterified.

9. Synthetic esters as claimed in claims 1 to 7, characterized in that the polybasic carboxylic acids are di- and/or tricarboxylic acids containing 2 to 54 carbon atoms.

10. Synthetic esters as claimed in claim 9, characterized in that the di- and/or tricarboxylic acids are succinic acid, adipic acid, sebacic acid, isomers of phthalic acid, dimer fatty acid or trimer fatty acid or mixtures thereof.

11. Synthetic esters as claimed in claims 9 and 10, characterized in that the equivalent OH:COOH ratio is in the range from 1.2:1 to 0.9:1 and preferably in the range from 1.05:1 to 0.95:1.

12. Synthetic esters as claimed in claims 9 to 11, characterized in that the ratio of COOH from polybasic carboxylic acids to COOH from monobasic carboxylic acids is in the range from 0.005 to 0.5:1, preferably in the range from 0.01 to 0.2:1 and more preferably in the range from 0.05 to 0.15:1.

13. Hydraulic oils containing synthetic esters of
a) fatty acid mixtures,
b) alcohols and
c) if desired, polybasic carboxylic acids,
characterized in that the fatty acid mixtures contain at least 85% by weight of oleic acid and 0.5 to 2.5% by weight of stearic acid, based on the mixture.

14. The use of the synthetic esters claimed in claims 1 to 7 and 9 to 12 as lubricants.

15. The use of the synthetic esters claimed in claims 1 to 7 and 9 to 12 as hydraulic oils.

16. The use of the synthetic esters claimed in claims 1 to 12 for cosmetic purposes, preferably as oils.

## Revendications

1. Esters synthétiques constitués
a) de mélanges d'acides gras
b) d'alcools, et
c) le cas échéant, d'acides carboxyliques polyfonctionnels,
caractérisés en ce que les mélanges d'acides gras renferment au moins 85 % en poids d'acide oléique et 0,5 à 2,5 % en poids d'acide stéarique (dans chaque cas par rapport au mélange).

2. Esters synthétiques selon la revendication 1, caractérisés en ce que la teneur en acide stéarique des mélanges d'acides gras est comprise entre 0,5 et 1,5 % en poids (par rapport au mélange).

3. Esters synthétiques selon les revendications 1 et 2, caractérisés en ce que du "high oleic, low stearic sunflower" (tournesol riche en acide oléique et pauvre en acide stéarique) est utilisé comme graine oléagineuse pour l'extraction des mélanges d'acides gras.

4. Esters synthétiques selon les revendications 1 à 3, caractérisés en ce que les alcools sont représentés par des polyols.

5. Esters synthétiques selon les revendications 1 à 4, caractérisés en ce que les polyols comportent 2 à 6 groupes hydroxyle.

6. Esters synthétiques selon les revendications 1 à 5, caractérisés en ce que les polyols ne présentent pas d'hydrogène sur l'atome de carbone, qui est voisin de l'atome de carbone portant le groupe hydroxyle.

7. Esters synthétiques selon les revendications 1 à 6, caractérisés en ce que les polyols sont représentés par le néopentylglycol, le triméthyloléthane, le triméthylolpropane, le ditriméthylolpropane, le pentaérythrite, le dipentaérythrite ou le tripentaérythrite ou par des mélanges de ceux-ci.

8. Esters synthétiques selon les revendications 4 à 7, caractérisés en ce que les esters des polyols représentent des esters partiels, dans lesquels 16 à 80 mol % en moyenne des groupes hydroxyle du polyol sont estérifiés.

9. Esters synthétiques selon les revendications 1 à 7, caractérisés en ce que les acides carboxyliques polyfonctionnels sont représentés par des acides di- et/ou tricarboxyliques comportant 2 à 54 atomes de C.

10. Esters synthétiques selon la revendication 9, caractérisés en ce que les acides di- et/ou tricarboxyliques sont représentés par l'acide succinique, adipique, sébacique, les isomères de l'acide phtalique, l'acide gras dimère ou trimère, ou les mélanges de ceux-ci.

11. Esters synthétiques selon les revendications 9 à 10, caractérisés en ce que le rapport des équivalents OH:COOH se situe dans l'intervalle de 1,2:1 à 0,9:1, de préférence dans la plage de 1,05:1 à 0.95:1.

12. Esters synthétiques selon les revendications 9 à 11, caractérisés en ce que le rapport des équivalents entre les groupes COOH d'acides carboxyliques polyfonctionnels et les groupes COOH d'acides carboxyliques monofonctionnels se situe dans l'intervalle de 0,005 à 0,5, de préférence de 0,01 à 0.02, avec une préférence particulière, dans la plage de 0,05 à 0,15.

13. Huiles hydrauliques renfermant des esters synthétiques constitués
a) de mélanges d'acides gras
b) d'alcools, et
c) le cas échéant, d'acides carboxyliques polyfonctionnels,
caractérisées en ce que les mélanges d'acides gras renferment au moins 85 % en poids d'acide oléique et 0,5 à 2,5 % en poids d'acide stéarique (dans chaque cas par rapport au mélange).

14. Utilisation des esters synthétiques selon les revendications 1 à 7 et 9 à 12 comme lubrifiants.

15. Utilisation des esters synthétiques selon les revendications 1 à 7 et 9 à 12 comme huiles hydrauliques.

16. Utilisation des esters synthétiques selon les revendications 1 à 12 à des usages cosmétiques, de préférence comme corps huileux.
